(19) Europäisches Patentamt
European Patent Office
Office européen des brevets

(11) **EP 2 558 142 B1**

(12) **EUROPÄISCHE PATENTSCHRIFT**

(45) Veröffentlichungstag und Bekanntmachung des Hinweises auf die Patenterteilung:
**17.08.2016 Patentblatt 2016/33**

(21) Anmeldenummer: **11719172.6**

(22) Anmeldetag: **14.04.2011**

(51) Int Cl.:
***A61M 1/16*** *(2006.01)*

(86) Internationale Anmeldenummer:
**PCT/EP2011/001891**

(87) Internationale Veröffentlichungsnummer:
**WO 2011/128098 (20.10.2011 Gazette 2011/42)**

(54) **VORRICHTUNG UND VERFAHREN ZUR ÜBERWACHUNG DES ANSCHLUSSES EINER BLUTBEHANDLUNGSEINHEIT AN DAS FLÜSSIGKEITSSYSTEM EINER EXTRAKORPORALEN BLUTBEHANDLUNGSVORRICHTUNG**

DEVICE AND METHOD FOR MONITORING THE CONNECTION OF A BLOOD TREATMENT UNIT TO THE LIQUID SYSTEM OF AN EXTRACORPOREAL BLOOD TREATMENT DEVICE

DISPOSITIF ET PROCÉDÉ POUR CONTRÔLER LE RACCORDEMENT D'UNE UNITÉ DE TRAITEMENT DU SANG AU SYSTÈME DE LIQUIDE D'UN DISPOSITIF DE TRAITEMENT EXTRACORPOREL DU SANG

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.07.2010 DE 102010032154**
**14.04.2010 DE 102010015003**

(43) Veröffentlichungstag der Anmeldung:
**20.02.2013 Patentblatt 2013/08**

(73) Patentinhaber: **Fresenius Medical Care Deutschland GmbH**
**61352 Bad Homburg v.d.H. (DE)**

(72) Erfinder:
• **HÄCKER, Jürgen**
**61267 Neu-Anspach (DE)**
• **GRONAU, Sören**
**64569 Nauheim (DE)**

(74) Vertreter: **Oppermann, Frank**
**OANDO Oppermann & Oppermann LLP**
**Washingtonstrasse 75**
**65189 Wiesbaden (DE)**

(56) Entgegenhaltungen:
**EP-A2- 1 078 642      WO-A1-2005/107833**
**DE-C1- 10 201 109**

**Beschreibung**

Die Erfindung betrifft eine Vorrichtung und ein Verfahren zur Überwachung des Anschlusses einer Blutbehandlungseinheit an das Flüssigkeitssystem einer extrakorporalen Blutbehandlungsvorrichtung, wobei die Blutbehandlungseinheit einen Einlass und einen Auslass zum Anschluss an das Flüssigkeitssystem und das Flüssigkeitssystem ein Leitungssystem mit einem ersten Leitungsabschnitt aufweist, der an den Einlass angeschlossen wird und einen zweiten Leitungsabschnitt aufweist, der an den Auslass der Blutbehandlungseinheit angeschlossen wird. Darüber hinaus betrifft die Erfindung eine extrakorporale Blutbehandlungsvorrichtung mit einer Vorrichtung zur Überwachung des Anschlusses der Blutbehandlungseinheit an das Flüssigkeitssystem der Blutbehandlungsvorrichtung, insbesondere das Dialysierflüssigkeitssystem der B lutbehandlungsvorrichtung.

Es sind verschiedene Arten von Blutbehandlungsvorrichtungen bekannt. Zu den bekannten Blutbehandlungsvorrichtungen zählen beispielsweise die Vorrichtungen zur Hämodialyse, Hämofiltration und Hämodiafiltration. Während der Blutbehandlung strömt das Blut des Patienten in einem extrakorporalen Blutkreislauf durch eine Blutbehandlungseinheit. Bei den Vorrichtungen zur Hämodialyse, Hämofiltration und Hämodiafiltration ist die Blutbehandlungseinheit ein Dialysator oder Filter, der durch eine semipermeable Membran in eine Blutkammer und eine Dialysierflüssigkeitskammer getrennt ist. Während der Blutbehandlung strömt das Blut durch die Blutkammer, während die Dialysierflüssigkeit durch die Dialysierflüssigkeitskammer strömt. Eine effektive Blutbehandlung setzt voraus, dass Blut und Dialysierflüssigkeit entlang der Membran des Dialysators oder Filters in entgegengesetzten Richtungen strömen. Bei gleicher Strömungsrichtung ist die Blutbehandlung weniger effektiv.

Der Dialysator oder Filter ist eine austauschbare Einheit, die mit dem Flüssigkeitssystem der Blutbehandlungsvorrichtung verbunden wird. Das Flüssigkeitssystem der bekannten Blutbehandlungsvorrichtungen umfasst ein Leitungssystem mit einem ersten und einem zweiten Leitungsabschnitt für den Anschluss der Blutbehandlungseinheit. Zum Anschluss des Dialysators oder Filters an das Flüssigkeitssystem wird der erste Leitungsabschnitt mit dem Einlass der Dialysierflüssigkeitskammer und der zweite Leitungsabschnitt mit dem Auslass der Dialysierflüssigkeitskammer des Dialysators verbunden. Der Anschluss des Dialysators erfolgt mit bekannten Anschlussstücken, zu denen die bekannten Hansenkupplungen zählen.

Die Hersteller von Dialysatoren und Blutbehandlungsvorrichtungen sehen eine farbliche Kodierung des Ein- und Auslasses des Dialysators sowie der mit dem Ein- und Auslass zu verbindenden Hansenkupplungen vor, um dem Anwender den ordnungsgemäßen Anschluss im Gegenstromprinzip zu erleichtern. Diese farbliche Kodierung ist allerdings nicht bei allen Herstellern einheitlich. Daher besteht die Gefahr, dass die Anschlüsse verwechselt werden, was nachfolgend als nicht ordnungsgemäßer Anschluss bezeichnet wird. Dies führt dazu, dass der Dialysator nicht im Gegenstrom betrieben wird. Folglich kann die Effektivität der Behandlung für den Patienten nicht ausreichend sein. Dies ist insofern problematisch, als ein nicht ordnungsgemäßer Anschluss des Dialysators möglicherweise unbemerkt bleibt. Daher besteht grundsätzlich die Gefahr, dass der Patient auf Dauer mit nicht ausreichender Effektivität behandelt wird.

Die DE 102 01 109 C1 beschreibt eine Vorrichtung zur Erkennung einer Leckage in einem Flüssigkeitssystem einer Blutbehandlungsvorrichtung. Die bekannte Vorrichtung sieht zunächst einen konventionellen Druckhaltetest vor, bei dem der Dialysator vom Flüssigkeitssystem getrennt ist. Darüber hinaus sieht die Vorrichtung eine fortlaufende Überwachung des Drucks in dem Flüssigkeitssystem vor, um aus der Änderung des Drucks die Leckrate in einem vorgegebenen Zeitintervall bestimmen zu können. Aus den Leckraten wird das Leckage-Volumen bestimmt, das mit einem vorgegebenen Grenzwert verglichen wird. Die Druckmessung im Flüssigkeitssystem erfolgt mit einem in der Dialysierflüssigkeitszuführ- und -abführleitung angeordneten Drucksensor.

Die US 2002/0121471 A1 beschreibt eine Blutbehandlungsvorrichtung mit einem Dialysator, der durch eine Membran in eine Blutkammer und eine Dialysierflüssigkeitskammer unterteilt ist. Die bekannte Dialysevorrichtung sieht die Bestimmung des sogenannten Transmembrandrucks TMP vor. Hierzu wird der Druck am Ein- und Auslass der Blutkammer und der Dialysierflüssigkeitskammer gemessen. Die Druckmessung erfolgt mit Drucksensoren an den Ein- und Auslässen des Dialysators. Die bekannte Blutbehandlungsvorrichtung sieht aber die Überwachung des korrekten Anschlusses der flüssigkeitsführenden Leitungen an den Dialysator nicht vor.

Die US 2005/0145549 A1 beschreibt eine Blutbehandlungsvorrichtung, bei der eine Umkehr des Blutflusses durch die Blutkammer des Dialysators vorgesehen ist. Die Überwachung des Anschlusses der Flüssigkeitsleitungen an den Dialysator sieht aber auch die US 2005/0145549 A1 nicht vor.

Der Erfindung liegt die Aufgabe zugrunde, eine Vorrichtung und ein Verfahren anzugeben, das die Sicherheit bei der Dialyse erhöht.

Die Lösung dieser Aufgabe erfolgt erfindungsgemäß mit den Merkmalen der unabhängigen Patentansprüche. Vorteilhafte Ausführungsformen der Erfindung sind Gegenstand der Unteransprüche.

Die erfindungsgemäße Vorrichtung und das erfindungsgemäße Verfahren zur Überwachung des Anschlusses einer Blutbehandlungseinheit an das Flüssigkeitssystem einer extrakorporalen Blutbehandlungsvorrichtung beruhen auf der Messung mindestens eines Drucks im Flüssigkeitssystem der Blutbehandlungsvorrichtung. Allein auf der Grundlage der mindestens einen Druckmessung wird festgestellt, ob der Anschluss der Blutbehandlungseinheit an das Flüssigkeitssystem ordnungsgemäß oder nicht ordnungsgemäß ist.

Die erfindungsgemäße Überwachungsvorrichtung sieht Mittel zum Messen des Drucks in dem ersten und/oder zweiten Leitungsabschnitt des Leitungssystems der Blutbehandlungsvorrichhtung vor. Grundsätzlich ist es möglich, den Druck nur in einem der beiden Leitungsabschnitte zu messen, um den korrekten Anschluss der Blutbehandlungseinheit an nur einen der beiden Leitungsabschnitte zu überwachen. Wenn der eine Leitungsabschnitt falsch angeschlossen ist, muss auch der andere Leitungsabschnitt falsch angeschlossen sein.

Es hat sich gezeigt, dass sich die statischen Druckverhältnisse der vollständig mit Flüssigkeit gefüllten Leitungsabschnitte mit der Höhenlage der Leitungsabschnitte im Raum ändern, während der Dialysator nicht mit Flüssigkeit gefüllt ist. Dies ist darauf zurück zu führen, dass bei den bekannten Blutbehandlungsvorrichtungen die Blutbehandlungseinheit und die daran anzuschließenden Leitungsabschnitte des Leitungssystems auf unterschiedlichen Höhen liegen.

Im Allgemeinen liegen Einlass und Auslass beider Kammern der Blutbehandlungseinheit nicht in einer gemeinsamen horizontalen Ebene, sondern übereinander. Bei den bekannten Blutbehandlungsvorrichtungen liegt der Einlass der Dialysierflüssigkeitskammer des Dialysators in Abhängigkeit vom Typ des Dialysators entweder oberhalb oder unterhalb des Auslasses der Dialysierflüssigkeitskammer des Dialysators. Wenn der Dialysator nicht ordnungsgemäß angeschlossen ist, ergeben sich andere Druckverhältnisse als bei einem ordnungsgemäßen Anschluss. Diese Veränderung der Druckverhältnisse ist die Grundlage für die Erkennung eines nicht ordnungsgemäßen Anschlusses.

Mit der erfindungsgemäßen Überwachungsvorrichtung und dem erfindungsgemäßen Überwachungsverfahren wird nicht die Integrität, insbesondere Dichtigkeit der Anschlüsse oder des Leitungssystems überprüft, sondern es wird überprüft, ob die Blutbehandlungseinheit korrekt an das Flüssigkeitssystem der Blutbehandlungsvorrichtung angeschlossen ist.

Bei einer bevorzugten Ausführungsform der Erfindung wird der Druck nicht nur in einem der beiden Leitungsabschnitte, sondern in beiden Leitungsabschnitten überwacht, um die Differenz zwischen dem in dem ersten Leitungsabschnitt und dem in dem zweiten Leitungsabschnitt gemessenen Druck zu bilden. Die Überwachung der Druckdifferenz hat den Vorteil, dass die Anordnung des Dialysators oder Filters in unterschiedlichen Höhen nicht zu unterschiedlichen Messergebnissen führt, da nicht eine absolute Größe, sondern nur eine relative Messgröße ausgewertet wird. Daher kann selbst dann der Anschluss der Blutbehandlungseinheit mit großer Sicherheit überwacht werden, wenn die Blutbehandlungseinheit in unterschiedlichen Höhen in die an den Blutbehandlungsvorrichtungen vorgesehenen Halterungen eingesetzt werden sollte.

Eine besonders bevorzugte Ausführungsform sieht vor, dass die Druckdifferenz zwischen dem Druck in dem ersten Leitungsabschnitt und dem Druck in dem zweiten Leitungsabschnitt mit einem vorgegebenen Grenzwert verglichen wird. Wenn die Druckdifferenz größer als der vorgegebene Grenzwert ist, wird auf einen ordnungsgemäßen Anschluss der Blutbehandlungseinheit geschlossen. Wenn die Druckdifferenz hingegen kleiner als der vorgegebene Grenzwert ist, wird auf einen nicht ordnungsgemäßen Anschluss geschlossen. Dies setzt aber voraus, dass der Einlass des Dialysators oberhalb des Auslasses liegt. Für den Fall, dass der Einlass unterhalb des Auslasses liegt, wird auf einen ordnungsgemäßen Anschluss der Blutbehandlungseinheit geschlossen, wenn die Druckdifferenz kleiner als der vorgegebene Grenzwert ist.

Für den Fall, dass der Anschluss der Blutbehandlungseinheit nicht ordnungsgemäß ist, wird vorzugsweise ein optischer und/oder akustischer und/oder taktiler Alarm gegeben. Darüber hinaus wird vorzugsweise die Durchführung der Blutbehandlung verhindert, so dass die Durchführung der Blutbehandlung nur bei ordnungsgemäßem Anschluss der Blutbehandlungseinheit möglich ist.

Die erfindungsgemäße Überwachungsvorrichtung und das erfindungsgemäße Überwachungsverfahren können bei den bekannten Blutbehandlungsvorrichtungen eingesetzt werden, ohne dass hierzu größere Umbaumaßnahmen erforderlich sind. Die Mittel zum Messen des Drucks in den beiden Leitungsabschnitten des Leitungssystems sind bei den bekannten Blutbehandlungsvorrichtungen ohnehin vorhanden. Auch sind bei den bekannten Blutbehandlungsvorrichtungen Absperrorgane in beiden Leitungsabschnitten vorhanden. Die Prüfung setzt aber voraus, dass die dialysatsseitigen Leitungen vollständig mit Flüssigkeit gefüllt sind, der Dialysator aber nicht mit Flüssigkeit gefüllt ist. Am Ende der bei den bekannten Blutbehandlungsvorrichtungen vorhandenen Dialysatorkupplungen, insbesondere Hansenkupplungen, herrscht somit Umgebungsdruck.

Die Überprüfung des Anschlusses der Blutbehandlungseinheit kann nach dem obligatorischen Spülvorgang erfolgen. Allerdings dürfen nur die zu der Blutbehandlungseinheit führenden und von der Blutbehandlungseinheit abgehenden Leitungsabschnitte des Flüssigkeitssystems, nicht aber die Blutbehandlungseinheit, insbesondere die Dialysierflüssigkeitskammer des Dialysators oder Filters, mit Flüssigkeit gefüllt sein. Zur Durchführung des Spülvorgangs ist das Leitungssystem des Flüssigkeitssystems der Blutbehandlungsvorrichtung vollständig mit Flüssigkeit befüllt. Die Aufnahme der Druckmesswerte erfolgt vorzugsweise nach dem Öffnen der Absperrorgane in den beiden Leitungsabschnitten des Leitungssystems.

Bei einer besonders bevorzugten Ausführungsform wirken die zentrale Steuereinheit der Blutbehandlungsvorrichtung und die Auswerteinheit der Überwachungsvorrichtung derart zusammen, dass nach Öffnen der Absperrorgane der Messvorgang automatisch abläuft.

Die erfindungsgemäße Vorrichtung und das erfindungsgemäße Verfahren erlauben nicht nur die sichere Überprüfung des Anschlusses der Blutbehandlungseinheit, sondern ermöglichen auch die vollständige Dokumentation, wenn das Ergebnis der Überprüfung gespeichert wird. Wenn die Behandlung nicht bei falschem Anschluss der Blutbehandlungseinheit verhindert wird, kann auch nach Durchführung der Behandlung, wenn die Blutbehandlungseinheit von dem Flüssigkeitssystem wieder getrennt ist, noch nachvollzogen werden, ob der Anschluss der Blutbehandlungseinheit ordnungsgemäß war. Wenn dies nicht der Fall war, kann der Arzt entsprechende Maßnahmen ergreifen.

Die oben beschriebenen Ausführungsformen dienen nur der Überwachung des Anschlusses der Blutbehandlungseinheit. Daher sollten die Leitungsabschnitte des Leitungssystems für den Benutzer möglichst einfach den entsprechenden Anschlüssen der Blutbehandlungseinheit zugeordnet werden können. Dies kann in bekannter Weise mit einer farblichen Kodierung erfolgen.

Bei einer weiteren bevorzugten Ausführungsform kann grundsätzlich darauf verzichtet werden, die Leitungsabschnitte des Leitungssystems den Anschlüssen der Blutbehandlungseinheit beispielsweise mit einer Farbkodierung zuzuordnen. Diese Ausführungsform sieht Mittel zum Umkehren der Flussrichtung vor, um bei einem nicht ordnungsgemäßen Anschluss, d.h. wenn die Blutbehandlungseinheit nicht im Gegenstrom betrieben würde, die Flussrichtung umzukehren, so dass die Blutbehandlungseinheit dann im Gegenstrom betrieben wird. Die Auswerteinheit der Überwachungsvorrichtung wirkt bei dieser Ausführungsform derart mit den Mitteln zum Umkehren der Flussrichtung zusammen, dass die Auswerteinheit die Mittel zum Umkehren der Flussrichtung aktiviert, wenn ein nicht ordnungsgemäßer Anschluss der Blutbehandlungseinheit an das Flüssigkeitssystem festgestellt wird. Auf diese Weise kann die ordnungsgemäße Strömungsrichtung, d.h. der Betrieb des Dialysators im Gegenstrom, hergestellt werden, ohne dass die Hansen-Kupplungen manuell neu angeschlossen werden müssten.

Es sei bemerkt, dass die Vorrichtung zur Umkehr der Flussrichtung auf der Dialysierflüssigkeitsseite von eigener erfinderischer Bedeutung ist. Daher kann die Vorrichtung zur Umkehr der Flussrichtung nicht nur bei der erfindungsgemäßen Vorrichtung zur Überwachung des Anschlusses der Blutbehandlungseinheit verwendet werden, sondern bei sämtlichen extrakorporalen Blutbehandlungsvorrichtungen, die neben dem extrakorporalen Blutkreislauf über ein Flüssigkeitssystem verfügen, das ein Leitungssystem mit einem ersten Leitungsabschnitt aufweist, der an den Einlass einer Blutbehandlungseinheit, insbesondere an den Einlass der Dialysierflüssigkeitskammer eines Dialysators oder Filters, angeschlossen wird, und einen zweiten Leitungsabschnitt aufweist, der an den Auslass der Blutbehandlungseinheit, insbesondere an den Auslass der Dialysierflüssigkeitskammer, angeschlossen wird. Die Blutbehandlungsvorrichtung zeichnet sich also durch Mittel zum Umkehren der Flussrichtung aus, die derart ausgebildet sind, dass in einer ersten Position der erste Leitungsabschnitt zu dem Einlass der Blutbehandlungseinheit und der zweite Leitungsabschnitt zu dem Auslass der Blutbehandlungseinheit führt, und dass in einer zweiten Position der erste Leitungsabschnitt zu dem Auslass der Blutbehandlungseinheit und der zweite Leitungsabschnitt zu dem Einlass der Blutbehandlungseinheit führt, wenn die Blutbehandlungseinheit an das Flüssigkeitssystem angeschlossen ist.

Im Folgenden wird ein Ausführungsbeispiel der Erfindung unter Bezugnahme auf die Zeichnungen näher erläutert.

Es zeigen:

Fig. 1     in stark vereinfachter schematischer Darstellung die wesentlichen Komponenten einer Blutbehandlungsvorrichtung,

Fig. 2A     eine schematische Darstellung der Anordnung der Blutbehandlungseinheit und der ordnungsgemäß an die Blutbehandlungseinheit angeschlossenen Leitungsabschnitte des Leitungssystems des Flüssigkeitssystems der Blutbehandlungsvorrichtung,

Fig. 2B     die nicht ordnungsgemäß an die Blutbehandlungseinheit angeschlossenen Leitungsabschnitte des Leitungssystems,

Fig. 3A     die Druckverhältnisse bei einem ordnungsgemäßen Anschluss der B lutbehandlungseinheit,

Fig. 3B     die Druckverhältnisse bei einem nicht ordnungsgemäßen Anschluss der Blutbehandlungseinheit

Fig. 4     in schematischer Darstellung die Leitungsabschnitte des Flüssigkeitssystems zusammen mit den Messpunkten der Drucksensoren,

Fig. 5     eine Teilansicht des Flüssigkeitssystems einer alternativen Ausführungsform der Blutbehandlungsvorrichtung in vereinfachter schematischer Darstellung, die über eine Vorrichtung zur Umkehr der Strömungsrichtung verfügt,

Fig. 6A eine weitere alternative Ausführungsform der Vorrichtung zur Umkehr der Strömungsrichtung in vereinfachter schematischer Darstellung vor der Umkehr der Flussrichtung,

Fig. 6B eine weitere alternative Ausführungsform der Vorrichtung zur Umkehr der Strömungsrichtung in vereinfachter schematischer Darstellung nach der Umkehr der Flussrichtung.

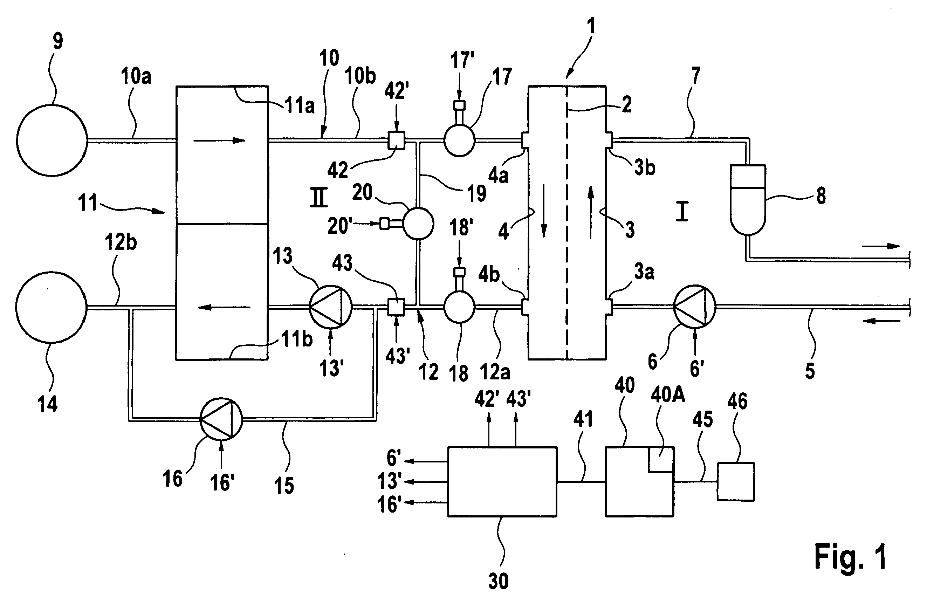

Fig. 1 zeigt in stark vereinfachter schematischer Darstellung die wesentlichen Komponenten einer Blutbehandlungsvorrichtung. Bei dem vorliegenden Ausführungsbeispiel ist die Blutbehandlungsvorrichtung eine Hämodialysevorrichtung, die einen Dialysator 1 aufweist, der durch eine semipermeable Membran 2 in eine Blutkammer 3 und eine Dialysierflüssigkeitskammer 4 getrennt ist. Der Einlass 3a der Blutkammer ist mit einem Ende der arteriellen Blutleitung 5 verbunden, in die eine Blutpumpe 6 geschaltet ist, während der Auslass 3b der Blutkammer mit einem Ende der venösen Blutleitung 7 verbunden ist, in die eine Tropfkammer 8 geschaltet ist. An den anderen Enden der arteriellen und venösen Blutleitung 5, 7 befinden sich die nicht dargestellten arteriellen und venösen Kanülen zum Anschluss an den Patienten. Dieser Teil der Blutbehandlungsvorrichtung stellt den extrakorporalen Blutkreislauf I dar.

Nachfolgend werden das erfindungsgemäße Verfahren und die erfindungsgemäße Vorrichtung am Beispiel einer Hämodialysevorrichtung beschrieben. Das erfindungsgemäße Verfahren und die erfindungsgemäße Vorrichtung können in gleicher Weise aber auch bei einer Hämodiafiltrationsvorrichtung Verwendung finden, da die Art der Dialysevorrichtung keinen Einfluss auf das erfindungsgemäße Verfahren und die erfindungsgemäße Vorrichtung hat.

Das Flüssigkeitssystem der Blutbehandlungsvorrichtung umfasst eine Einrichtung 9 zur Bereitstellung frischer Dialysierflüssigkeit, die über einen ersten Abschnitt 10a einer Dialysierflüssigkeitszuführleitung 10 mit dem Einlass einer Kammerhälfte 11a einer Bilanziereinrichtung 11 verbunden ist. Der zweite Abschnitt 10b der Dialysierflüssigkeitszuführleitung 10 verbindet den Auslass der einen Bilanzierkammerhälfte 11a mit dem Einlass 4a der Dialysierflüssigkeitskammer 4. Der Auslass 4b der Dialysierflüssigkeitskammer 4 ist über den ersten Abschnitt 12a der Dialysierflüssigkeitsabführleitung 12 mit dem Einlass der anderen Bilanzierkammerhälfte 11b der Bilanziereinrichtung 11 verbunden. In den ersten Abschnitt 12a der Dialysierflüssigkeitsabführleitung 12 ist eine Dialysierflüssigkeitspumpe 13 geschaltet. Der Auslass der anderen Bilanzierkammerhälfte 11b ist über den zweiten Abschnitt 12b der Dialysierflüssigkeitsabführleitung 12 mit einem Ablauf 14 verbunden. Stromauf der Dialysierflüssigkeitspumpe 13 zweigt von der Dialysierflüssigkeitsabführleitung 12 eine Ultrafiltratleitung 15 ab, die ebenfalls zu dem Ablauf 14 führt. In die Ultrafiltratleitung 15 ist eine Ultrafiltrationspumpe 16 geschaltet.

Während der Blutbehandlung wird die Blutkammer 3 von dem Blut des Patienten und die Dialysierflüssigkeitskammer 4 des Dialysators 1 von der Dialysierflüssigkeit durchströmt. Blut und Dialysierflüssigkeit strömen dabei entlang der Membran 2 des Dialysators 1 in entgegengesetzter Richtung. Dies setzt einen ordnungsgemäßen Anschluss der Blutbehandlungseinheit 1 an das Flüssigkeitssystem II bzw. den extrakorporalen Blutkreislauf I voraus.

Die Blutbehandlungsvorrichtung verfügt über eine zentrale Steuereinheit 30, die über Steuerleitungen 6', 13', 16' mit der Blutpumpe 6, der Dialysierflüssigkeitspumpe 13 und der Ultrafiltrationspumpe 16 verbunden ist.

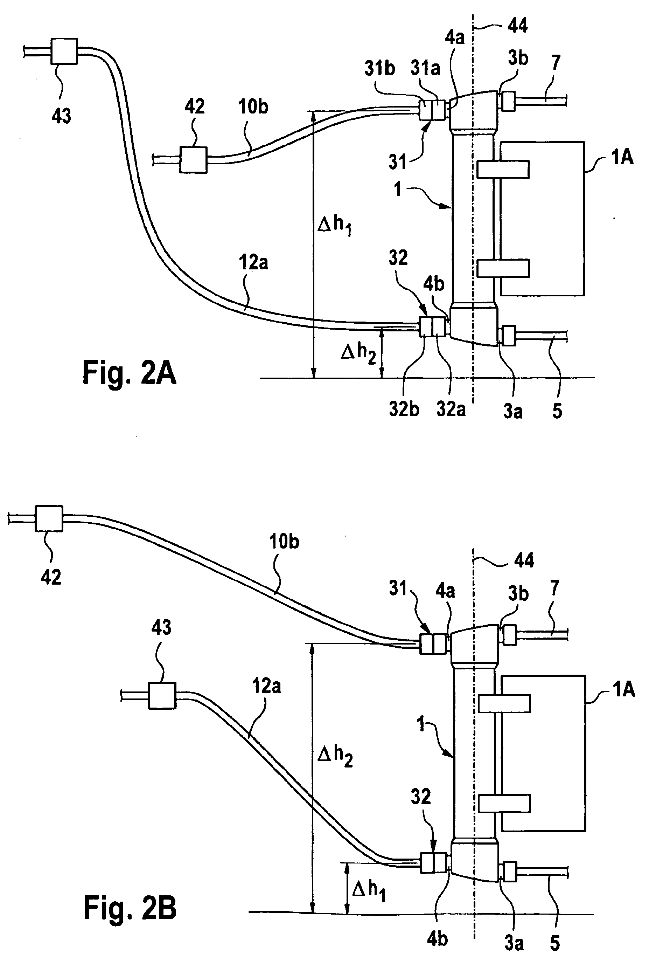

Die Dialysierflüssigkeitszuführ- und Dialysierflüssigkeitsabführleitung 10, 12 bilden ein Teil des Leitungssystems des Flüssigkeitssystems II der Blutbehandlungsvorrichtung. Der zu der Blutbehandlungseinheit 1 führende Leitungsabschnitt 10b des Flüssigkeitssystems II und der von der Blutbehandlungseinheit 1 abgehende Leitungsabschnitt 12a des Flüssigkeitssystems sind Schlauchleitungen, die an den Ein- und Auslass 4a, 4b der Dialysierflüssigkeitskammer 4 des Dialysators 1 angeschlossen werden. Dieser Teil des Flüssigkeitssystems wird unter Bezugnahme auf die Fig. 2A und 2B nachfolgend noch näher beschrieben.

Zum Abtrennen der Blutbehandlungseinheit 1 von dem Flüssigkeitssystem II befinden sich in der Dialysierflüssigkeitszuführleitung 10 ein erstes Absperrorgan 17 und in der Dialysierflüssigkeitsabführleitung 12 ein zweites Absperrorgan 18. Stromauf des ersten Absperrorgans 17 zweigt von der Dialysierflüssigkeitszuführleitung 10 eine Bypassleitung 19 ab, die zu der Dialysierflüssigkeitsabführleitung 12 stromab des zweiten Absperrorgans 18 führt. In die Bypassleitung 19 ist ein drittes Absperrorgan 20 geschaltet. Bei den Absperrorganen 17, 18, 20 handelt es sich um vorzugsweise elektromagnetisch betätigbare Absperrorgane, die über Steuerleitungen 17', 18', 20' von der zentralen Steuereinheit 30 angesteuert werden.

Nachfolgend wird die Vorrichtung zur Überwachung des ordnungsgemäßen Anschlusses der Blutbehandlungseinheit 1 an das Flüssigkeitssystem II der Blutbehandlungsvorrichtung im Einzelnen beschrieben.

Die Überwachungsvorrichtung kann eine selbständige Einheit bilden, aber auch Bestandteil der Blutbehandlungsvorrichtung sein. Da die bekannten Blutbehandlungsvorrichtungen bereits über Komponenten verfügen, von denen auch die Überwachungsvorrichtung Gebrauch macht, ist die Überwachungsvorrichtung vorzugsweise Bestandteil der Blutbehandlungsvorrichtung.

Die Überwachungsvorrichtung weist eine Rechen- und Auswerteinheit 40 auf, die in Fig. 1 dargestellt ist. Über eine Datenleitung 41 ist die Rechen- und Auswerteinheit 40 mit der zentralen Steuereinheit 30 der Blutbehandlungsvorrichtung verbunden. Die Rechen- und Auswerteinheit 40 kann aber auch Bestandteil der zentralen Steuereinheit 30 sein.

Die Überwachungsvorrichtung weist einen ersten Drucksensor 42 auf, der den Druck in der Dialysierflüssigkeitszuführleitung 10 stromauf des ersten Absperrorgans 17 misst. Darüber hinaus weist die Auswerteinheit 40 einen zweiten Drucksensor 43 auf, die den Druck in der Dialysierflüssigkeitsabführleitung 12 stromab des zweiten Absperrorgans 18 misst. Beide Drucksensoren 42,43 sind über Datenleitungen 42', 43' mit der Rechen- und Auswerteinheit 40 verbunden, so dass die Rechen- und Auswerteinheit 40 die Messwerte der Drucksensoren auswerten kann.

Fig. 2A und Fig. 2B zeigen den Teil des Flüssigkeitssystems der Blutbehandlungsvorrichtung, der die zu der Blutbehandlungseinheit 1 führenden Leitungsabschnitte 10b, 12a des Leitungssystems umfasst. Der besseren Übersichtlichkeit halber sind die Bypassleitung und die Absperrorgane nicht dargestellt.

Die Blutbehandlungseinheit, insbesondere der Dialysator 1, wird in eine nur schematisch dargestellte Halterung 1A der Blutbehandlungsvorrichtung eingesetzt. In der Halterung 1A ist der Dialysator derart angeordnet, dass Ein- und Auslass 4a, 4b des Dialysators auf unterschiedlichen Höhen liegen. Bei dem Ausführungsbeispiel ist die Längsachse 44 des Dialysators 1 in vertikaler Richtung angeordnet, wobei der Einlass 4a oben und der Auslass 4b der Dialysierflüssigkeitskammer 4 unten liegt. Die Höhe des Einlasses 4a und die Höhe des Auslasses 4b in Bezug auf eine Bezugs-höhe sind mit $\Delta h_1$ und $\Delta h_2$ bezeichnet.

Vor der Durchführung der Blutbehandlung wird die Blutbehandlungsvorrichtung in bekannter Weise gespült und entlüftet. Die Dialysierflüssigkeitskammer 4 des Dialysators 1 wird vor der Überprüfung des ordnungsgemäßen Anschlusses des Dialysators nicht mit Flüssigkeit gefüllt. Ansonsten wird das Flüssigkeitssystem mit Flüssigkeit gefüllt. Um die Dialysierflüssigkeitszuführ- und -abführleitungen 10, 12 vollständig mit Flüssigkeit füllen zu können, können die Leitungen zum Zeitpunkt des Spülens beispielsweise mit einem nicht dargestellten Kurzschlussstück an ihren Enden kurzgeschlossen werden. Zum Spülen werden die Absperrorgane 17 und 18 in den Dialysierflüssigkeitszuführ- und -abführleitungen 10, 12 geöffnet und das Absperrorgan 20 in der Bypassleitung 19 wird geschlossen.

Nunmehr wird die Blutbehandlungseinheit 1 an die Leitungsabschnitte 10b, 12a der Dialysierflüssigkeitszuführ-und -abführleitungen 10, 12 angeschlossen. Zum Anschluss der Blutbehandlungseinheit werden die Absperrorgane 17 und 18 in den Dialysierflüssigkeitszuführ- und -abführleitungen 10, 12 wieder geschlossen und das Kurzschlussstück entfernt. Nach dem Anschluss der Blutbehandlungseinheit können die Absperrorgane 17 und 18 wieder geöffnet und das Absperrorgan 20 geschlossen werden.

Der Anschluss der Leitungsabschnitte an die Blutbehandlungseinheit erfolgt mit bekannten Anschlussstücken 31, 32, insbesondere Hansenkupplungen. Die entsprechenden Kupplungsstücke der Hansenkupplung sind farblich kodiert, beispielsweise sind das dialysatorseitige Kupplungsstück 31 a des Einlasses 4a und das an den Einlass anzuschließende leitungsseitige Kupplungsstück 31b der Hansenkupplung 31 rot gekennzeichnet, während das dialysatorseitige Kupplungsstück 32a des Auslasses 4b und das entsprechende leitungsseitige Kupplungsstück 32b blau gekennzeichnet sind.

Nach dem Anschluss der Blutbehandlungseinheit 1 an das Flüssigkeitssystem II schließt die Steuereinheit das Absperrorgan 20 in der Bypassleitung 19 und öffnet das erste und zweite Absperrorgan 17 und 18 in den Zu- und -abführleitungen 10, 12, wobei die Dialysierflüssigkeitskammer 4 nicht mit Flüssigkeit gefüllt ist. Nach dem Öffnen beider Absperrorgane erfasst die Rechen- und Auswerteinheit 40 mit dem ersten und zweiten Drucksensor 42, 43 den Druck in dem Leitungsabschnitt 10b der Zuführleitung 10 und in dem Leitungsabschnitt 12a der Abführleitung 12. Die Druckmessung sollte nach Ablauf einer kurzen Verzögerungszeit erfolgen, so dass Einschwingvorgänge aufgrund des Umschaltens der Absperrorgane abgeklungen sind.

Die Rechen- und Auswerteinheit 40 berechnet nunmehr die Differenz von dem Druck im Zulauf, der mit dem ersten Drucksensor 42 gemessen wird, und dem Druck im Ablauf, der mit dem zweiten Drucksensor 43 gemessen wird. Den Betrag der berechneten Druckdifferenz $\Delta p$ vergleicht die Rechen- und Auswerteinheit 40 mit einem vorgegebenen Grenzwert. Wenn der Betrag der Druckdifferenz größer als der Grenzwert ist, stellt die Rechen- und Auswerteinheit 40 einen ordnungsgemäßen Anschluss der Blutbehandlungseinheit an das Flüssigkeitssystem II fest. Wenn der Betrag der Druckdifferenz hingegen kleiner oder gleich dem Grenzwert ist, stellt die Rechen- und Auswerteinheit einen nicht ordnungsgemäßen Anschluss fest. Dies setzt aber voraus, dass der Einlass 4a des Dialysators 1 oberhalb des Auslasses 4b liegt. Für den Fall, dass der Einlass unterhalb des Auslasses liegt, wird auf einen ordnungsgemäßen Anschluss der Blutbehandlungseinheit geschlossen, wenn die Druckdifferenz kleiner als ein vorgegebener Grenzwert ist. Bei dem vorliegenden Ausführungsbeispiel liegt aber der Einlass des Dialysators über dem Auslass.

Die Rechen- und Auswerteinheit 40 erzeugt bei einem nicht ordnungsgemäßen Anschluss ein Steuersignal, das die zentrale Steuereinheit 30 über die Datenleitung 41 empfängt. Die zentrale Steuereinheit 30 nimmt nunmehr einen Eingriff in die Maschinensteuerung vor. Dieser Eingriff kann darin liegen, dass die Durchführung der Blutbehandlung verhindert wird. Die Auswert- und Recheneinheit 40 ist über eine Datenleitung 45 mit einer Alarmeinheit 46 verbunden, die für den Fall eines nicht ordnungsgemäßen Anschlusses einen optischen und/oder akustischen und/oder taktilen Alarm gibt. Beispielsweise kann auf einer Anzeigeeinheit der Blutbehandlungsvorrichtung, insbesondere einem Bildschirm, eine Fehlermeldung mit einem Hinweis auf die Fehlerursache erfolgen.

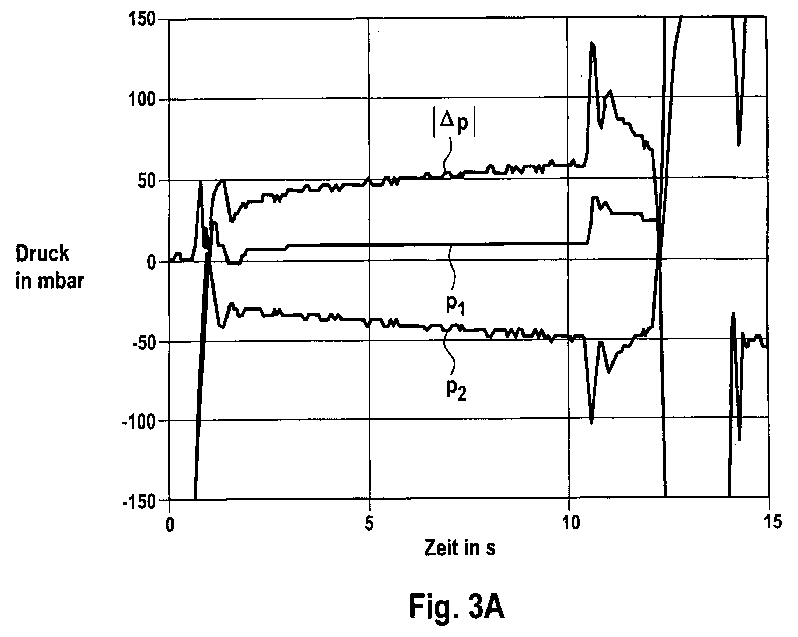

Fig. 3A zeigt die Druckverhältnisse bei einem ordnungsgemäßen Patientenanschluss, der in Fig. 2A dargestellt ist. Es zeigt sich, dass der Druck $p_1$ im Zulauf nach dem Öffnen der Absperrorgane 17, 18 ansteigt, während der Druck

$p_2$ im Ablauf abfällt. Es ergibt sich eine Druckdifferenz $\Delta p = p_1 - p_2$. Zur Bestimmung der Druckdifferenz ist eine einmalige Druckmessung zu einem bestimmten Zeitpunkt nach Öffnen der Absperrorgane grundsätzlich ausreichend. Es können auch mehrere Druckmessungen mit den Sensoren 42, 43 vorgenommen und die Messwerte statistisch ausgewertet werden. Beispielsweise kann der Mittelwert aus mehreren Druckmessungen gebildet werden. Es zeigt sich, dass der Betrag der Druckdifferenz $\Delta p = p_1 - p_2$ zwischen 40 und 60 mbar liegt. Die Rechen- und Auswerteinheit berechnet bspw. den Mittelwert, der bei 50 mbar liegt.

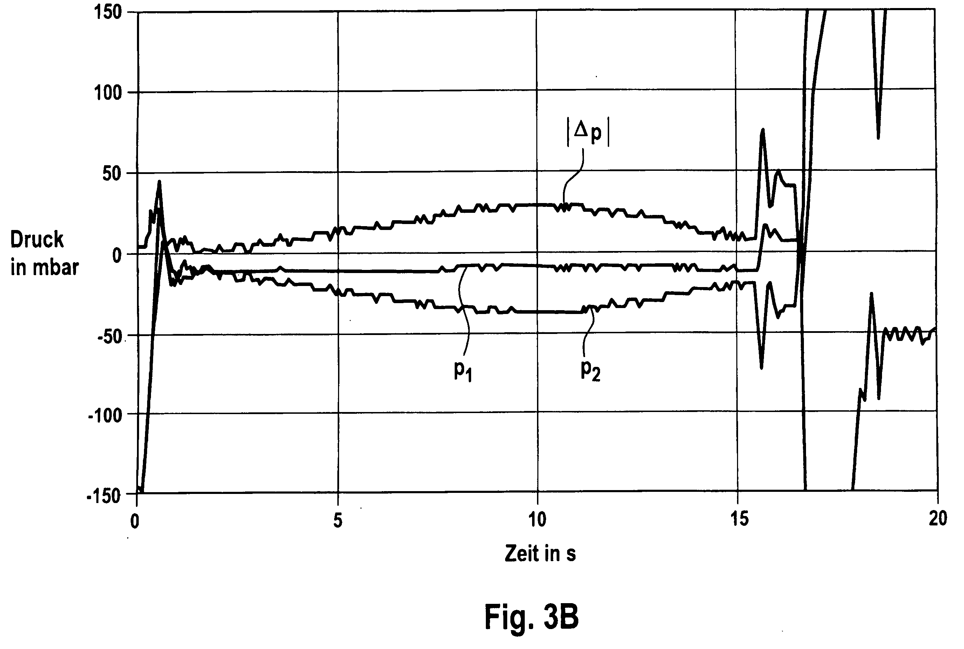

Fig. 3B zeigt die Druckverhältnisse bei einem nicht ordnungsgemäßen Anschluss (Fig. 2B). Der Betrag der Druckdifferenz $\Delta p = p_1 - p_2$ beträgt maximal 30 mbar.

Bei dem vorliegenden Ausführungsbeispiel ist der vorgegebene Grenzwert 40 mbar. Die Rechen- und Auswerteinheit 40 vergleicht den Betrag der maximalen Druckdifferenz $\Delta p$ von 50 mbar mit dem vorgegebenen Grenzwert von 40 mbar. Da der Betrag der Druckdifferenz $\Delta p$ größer als der vorgegebene Grenzwert ist, stellt die Rechen- und Auswerteinheit 40 den ordnungsgemäßen Anschluss der Blutbehandlungseinheit an das Flüssigkeitssystem fest (Fig. 2A und Fig. 3A). Wenn hingegen für den Betrag der Druckdifferenz ein Wert von 30 mbar berechnet wird, stellt die Rechen- und Auswerteinheit 40 einen nicht ordnungsgemäßen Anschluss fest (Fig. 2B und Fig. 3B), da der Betrag der Druckdifferenz kleiner als der vorgegebene Grenzwert ist.

Die unterschiedlichen Druckverhältnisse für den ordnungsgemäßen bzw. nicht ordnungsgemäßen Anschluss ergeben sich aus den unterschiedlichen statischen Drücken der flüssigkeitsgefüllten Schlauchleitungsabschnitte. Es zeigt sich, dass die Anordnung der Enden der Schlauchleitungsabschnitte 10b, 12a auf unterschiedlichen Höhen $\Delta h_1$ bzw. $\Delta h_2$ bezüglich der Bezugsebene zu unterschiedlichen Drücken führt. Es bilden sich in den Leitungsabschnitten 10b, 12a zwei "Flüssigkeitssäulen" aus.

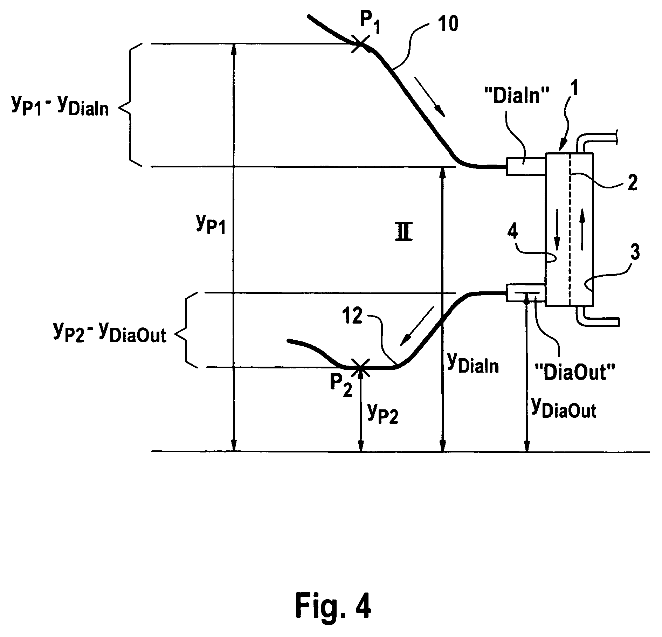

Fig. 4 zeigt zur weiteren Veranschaulichung in schematischer Darstellung die zu der Dialysierflüssigkeitskammer 4 des Dialysators 1 führenden und von der Dialysierflüssigkeitskammer 4 des Dialysators 1 abgehenden Leitungsabschnitte 10, 12 des Flüssigkeitssystems II zusammen mit den Messpunkten des ersten und zweiten Drucksensors 42,43 (Fig. 1), wobei der erste Drucksensor am Messpunkt P1 und der zweite Sensor am Messpunkt P2 angeordnet sind. Beide Drucksensoren sitzen am Ende einer vollständig gefüllten, volumenstarren, einseitig verschlossenen Leitung, die am anderen Ende gegen die Umgebung geöffnet ist. Der hydrostatische Druck am Drucksensor P1 ergibt sich somit aus der Höhendifferenz zum Dialysatoranschluss, gleiches gilt für den hydrostatischen Druck am Drucksensor P2.

Seien die Höhen der Drucksensoren und der Dialysatoranschlüsse bekannt:

| | |
|---|---|
| Höhe des ersten Drucksensors: | $y_{P1}$ |
| Höhe des zweiten Drucksensors: | $y_{P2}$ |
| Höhe des Dialysatzulaufs am Dialysator: | $y_{DiaIn}$ |
| Höhe des Dialysatrücklaufs am Dialysator: | $y_{DiaOut}$ |

Dann ergibt sich für einen Anschluss im Gegenstromprinzip folgende Höhendifferenz:

$$\Delta y_{Counterflow} = (y_{P1} - y_{DiaIn}) - (y_{P2} - y_{DiaOut})$$

Im Fall der Vertauschung der Anschlüsse ergibt sich:

$$\Delta y_{Coflow} = (y_{P1} - y_{DiaOut}) - (y_{P2} - y_{DiaIn})$$

Der Höhenunterschied zwischen der richtigen und der falschen Filterkonnektion ist:

$$\Delta y = \Delta y_{Counterflow} - \Delta y_{Coflow}$$

$$\Delta y = [(y_{P1} - y_{DiaIn}) - (y_{P2} - y_{DiaOut})] - [(y_{P1} - y_{DiaOut}) - (y_{P2} - y_{DiaIn})]$$
$$= y_{P1} - y_{DiaIn} - y_{P2} + y_{DiaOut} - y_{P1} + y_{DiaOut} + y_{P2} - y_{DiaIn}$$
$$= -y_{DiaIn} + y_{DiaOut} + y_{DiaOut} - y_{DiaIn}$$
$$= 2(y_{DiaOut} - y_{DiaIn})$$

Die Position der Drucksensoren spielt also keine Rolle, nur der Abstand zwischen Zu- und Ablauf am Dialysator geht in die Formel ein.

Die Höhendifferenzen sind proportional zum Druck:

$$\Delta y \sim \Delta p \rightarrow \Delta p_{Konnektion} \sim 2(y_{DiaOut} - y_{DiaIn})$$

Gleichung (1)

Bei dem vorliegenden Ausführungsbeispiel beträgt der Abstand zwischen dem Zu- und Ablauf 25 cm. Nach Gleichung (1) ergibt sich somit ein hydrostatischer Druckunterschied zwischen richtigem und falschem Anschluss des Dialysators oder Filters von ca. 50 mbar.

In der Praxis kann der Flussplan des Flüssigkeitssystems komplexer sein. Der hydraulische Druck am jeweiligen Drucksensor ergibt sich aus der besonderen Ausbildung des Flüssigkeitssystems einerseits und der Art des Anschlusses des Dialysators oder Filters andererseits. Da das Flüssigkeitssystem aber immer den gleichen Einfluss auf den hydrostatischen Druck hat, kann eine Abweichung der mit den Drucksensoren an den Messpunkten P1 und P2 gemessenen Druckdifferenz vom Sollwert nur mit einem falschen Anschluss des Dialysators oder Filters erklärt werden. Voraussetzung ist, dass der Zustand der Hydraulik zum Messzeitpunkt definiert ist, was unmittelbar nach dem Anschluss des Dialysators oder Filters der Fall ist (Fig. 3A und Fig. 3B).

Innerhalb eines vorgegebenen Zeitintervalls nach dem Anschluss des Dialysators oder Filters an die Leitungsabschnitte des Flüssigkeitssystems wird die Druckdifferenz an den Messpunkten P1 und P2 ermittelt:

$$\Delta p_{mess} = P1 - P2$$

Dieser Wert wird mit dem Sollwert $\Delta p_{system}$ verglichen. Der Sollwert $\Delta p_{system}$ wird für die Blutbehandlungsvorrichtung empirisch ermittelt. Es ist möglich, für mehrere Blutbehandlungsvorrichtungen des gleichen Typs nur einen empirisch ermittelten Wert heranzuziehen. Mit dem nach Gleichung (1) berechneten Wert $\Delta p_{Konnektion}$ wird die Schwelle definiert, ab der auf einen nicht ordnungemäßen Anschluss des Dialysators oder Filters geschlossen wird.

Wenn die Druckdifferenz zwischen P1 und P2 kleiner ist als der Sollwert abzüglich einer vorgegebenen Toleranz, wird auf einen nicht ordnungsgemäßen Anschluss des Dialysators oder Filters geschlossen.

$$\Delta p_{mess} < \Delta p_{System} - f \cdot \Delta p_{Konnektion}$$

Beispiel:

| | |
|---|---|
| $\Delta p_{System}$ | = 50mbar (empirisch ermittelt) |
| $\Delta p_{Konnektion}$ | = 50mbar (aufgrund des minimalen Abstands zwischen dem Dialysatorzulauf am Dialysator und dem Dialysatorablauf am Dialysator) |
| $f$ | = 1/2 |

(fortgesetzt)

$$\Leftrightarrow \Delta p_{mess} \qquad < 25 \text{ mbar}$$

Somit wird ab einem $\Delta p_{mess}$ kleiner als 25 mbar ein nicht korrekter Anschluss des Dialysators gemeldet.

Die zentrale Steuereinheit 30 oder die Rechen- und Auswerteinheit 40 verfügen über einen Speicher 40A, in dem das Ergebnis der Überprüfung des Anschlusses gespeichert wird. Damit wird der ordnungsgemäße oder nicht ordnungsgemäße Anschluss protokolliert. Der Speicherinhalt kann auf einem nicht dargestellten Display der Blutbehandlungsvorrichtung dargestellt werden oder kann ausgelesen werden.

In der Praxis ist nur die Überwachung der Anschlüsse der Blutbehandlungseinheit 1 an das Leitungssystem des Flüssigkeitssystems II erforderlich. Auf der Blutseite besteht die Gefahr von Verwechselungen der Anschlüsse in der Praxis hingegen nicht. Rein theoretisch, wenn auch nicht ohne weiteres praktikabel, wäre es aber auch möglich, die erfindungsgemäße Vorrichtung zur Überwachung des Anschlusses der Blutbehandlungseinheit 1 an die Schlauchleitungen 5, 7 des extrakorporalen Blutkreislaufs II vorzusehen, um überwachen zu können, dass die blutseitigen Anschlüsse korrekt sind.

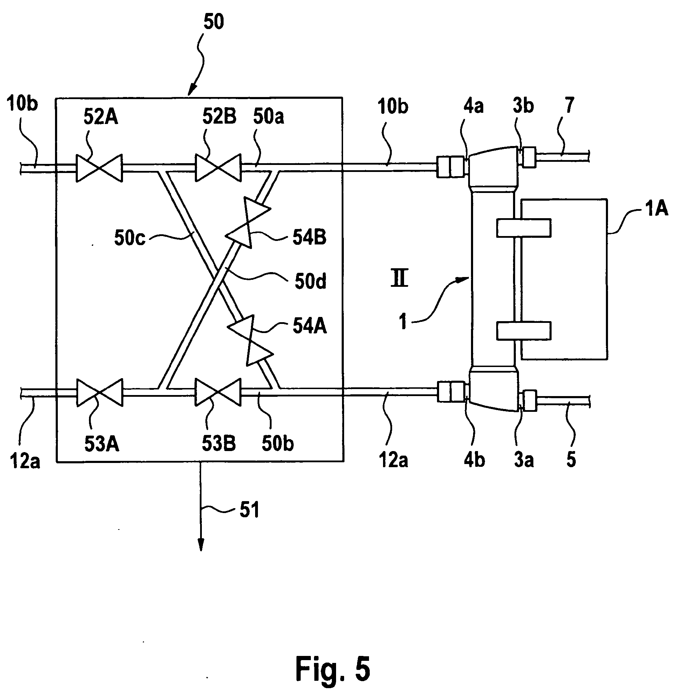

Fig. 5 zeigt einen Teil des Flüssigkeitssystems II einer alternativen Ausführungsform der erfindungsgemäßen Blutbehandlungsvorrichtung. Die einander entsprechenden Teile sind mit den gleichen Bezugszeichen versehen. Die alternative Ausführungsform unterscheidet sich von dem zuvor beschriebenen Ausführungsbeispiel dadurch, dass in die Leitungsabschnitte 10b, 12a der Dialysierflüssigkeitszuführ- und -abführleitung 10, 12 Mittel 50 zum Umkehren der Flussrichtung geschaltet sind, die in Fig. 4 nur schematisch dargestellt sind. Die Mittel 50 zum Umkehren der Flussrichtung sind über eine Datenleitung 51 mit der Steuer- und Recheneinheit 40 der Überwachungsvorrichtung verbunden. Wenn die zentrale Steuereinheit 30 der Blutbehandlungsvorrichtung das Steuersignal der Rechen- und Auswerteinheit 40 empfängt, das einen nicht ordnungsgemäßen Anschluss der Blutbehandlungseinheit signalisiert, steuert die zentrale Steuereinheit 30 die Mittel 50 zum Umkehren der Flussrichtung derart an, dass die Flussrichtung umgekehrt wird. Bei dieser Ausführungsform können die Hansenkupplungen grundsätzlich beliebig angeschlossen werden, da nach dem Feststellen eines nicht ordnungsgemäßen Anschlusses die Flussrichtung automatisch umgekehrt wird, so dass die Blutbehandlungseinheit im Gegenstrom betrieben wird.

Die Mittel 50 zum Umkehren der Flussrichtung weisen einen ersten Leitungsabschnitt 50a auf, der in den Leitungsabschnitt 10b der Dialysierflüssigkeitszuführleitung 10 geschaltet ist, und einen zweiten Leitungsabschnitt 50b, der in den Leitungsabschnitt 12a der Dialysierflüssigkeitsabführleitung 12 geschaltet ist. In den ersten und zweiten Leitungsabschnitt 50a, 50b sind jeweils zwei von der zentralen Steuereinheit 30 betätigbare Absperrorgane 52A, 52B bzw. 53A, 53B geschaltet. Von dem zwischen den Absperrorganen 52A, 52B liegenden Leitungsabschnitt geht eine erste Verbindungsleitung 50c ab, die zu dem Leitungsabschnitt zwischen dem Dialysator 1 und dem dritten Absperrorgan 53B führt. Von dem zwischen den Absperrorganen 53A, 53B liegenden Leitungsabschnitt geht eine zweite Verbindungsleitung 50d ab, die zu dem Leitungsabschnitt zwischen dem zweiten Absperrorgan 52B und dem Dialysator 1 führt. In die erste Verbindungsleitung 50c ist ein fünftes Absperrorgan 54A und in die Verbindungsleitung 50d ein sechstes Absperrorgan 54B geschaltet.

Zunächst sind das vierte und fünfte Absperrorgan 54A und 54B geschlossen und die anderen Absperrorgane 52A, 52B und 53A, 53B geöffnet, so dass die Flussrichtung nicht umgekehrt ist. Für das Umkehren der Flussrichtung wird das zweite und vierte Absperrorgan 52B, 53B geschlossen und das fünfte und sechste Absperrorgan 54A, 54B geöffnet. Das Öffnen und Schließen der jeweiligen Absperrorgane erfolgt automatisch von der Steuereinheit 30 in Abhängigkeit von dem Ergebnis der Überprüfung des Anschlusses der Blutbehandlungseinheit. Zur Erhöhung der Sicherheit kann nach der Umkehr der Flussrichtung eine erneute Überprüfung des ordnungsgemäßen Anschlusses der Blutbehandlungseinheit 1 mit der Überwachungsvorrichtung erfolgen. Bei dem vorliegenden Ausführungsbeispiel können die Absperrorgane 52A und 52B in der Dialysierflüssigkeitszuführ- und -abführleitung 10 und 12 auch entfallen.

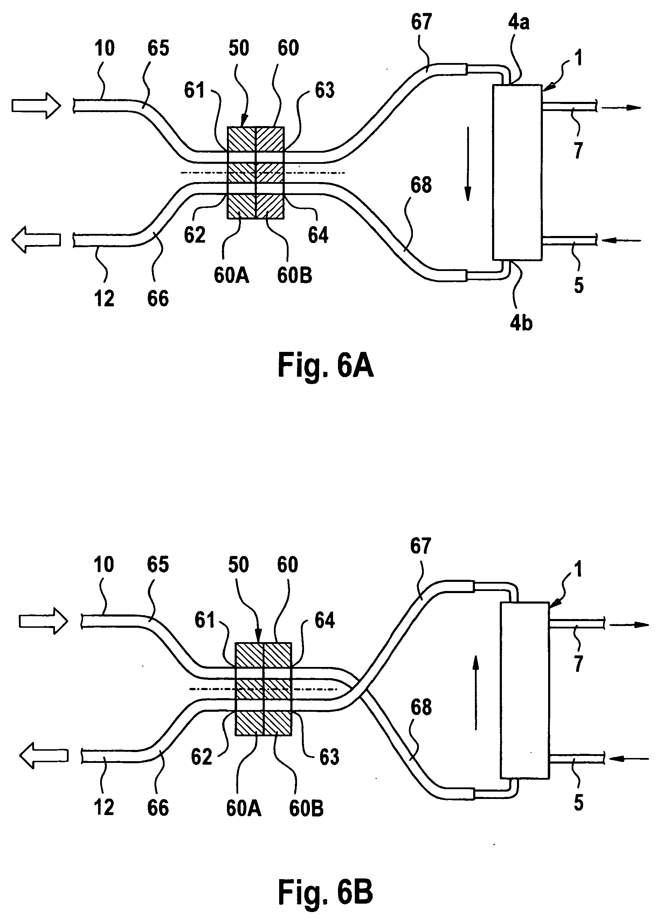

Die Fig. 6A und Fig. 6B zeigen eine weitere alternative Ausführungsform der Vorrichtung zur Umkehr der Flussrichtung in schematischer Darstellung, die sich von dem Ausführungsbeispiel von Fig. 5 dadurch unterscheidet, dass anstelle von einzelnen Absperrorganen in den Zu- und -ablaufleitungen 10, 12 ein gemeinsames Umschaltventil vorgesehen ist. Für die einander entsprechenden Teile werden wieder die gleichen Bezugszeichen verwendet. Die Fig. 6A und 6B zeigen die Vorrichtung 50 zur Umkehr der Flussrichtung nur in schematischer Darstellung. Das Umschaltventil 60 weist einen ersten und einen zweiten Ventilkörper 60A, 60B auf, die jeweils einen ersten und einen zweiten Anschluss 61, 62; 63, 64 haben. Der erste Anschluss 61 des ersten Ventilkörpers 60A ist mit einem zu dem ersten Ventilkörper führenden Abschnitt 65 der Dialysierflüssigkeitsleitung 10 verbunden, während der zweite Anschluss 62 des ersten Ventilkörpers 60A mit einem von dem ersten Ventilkörper 60A abgehenden Abschnitt 66 der Dialysierflüssigkeitsleitung 12 verbunden ist. Der erste Anschluss 63 des zweiten Ventilkörpers 60B ist mit einem von dem zweiten Ventilkörper abgehenden und zu dem einem Anschluss 4a der Blutbehandlungseinheit 1 führenden Abschnitt 67 der Dialysierflüssigkeitsleitung 10 verbunden, während der zweite Anschluss 64 des zweiten Ventilkörpers 60B mit einem von dem anderen Anschluss 4b der Blutbehandlungseinheit 1 abgehenden und zu dem zweiten Ventilkörper führenden Abschnitt 68 der Dialysierflüssigkeitsleitung 12 verbunden ist.

Der erste und zweite Ventilkörper 60A, 60B können derart gegeneinander verdreht werden, dass in einer ersten Position (Fig. 6A) eine Strömungsverbindung zwischen den ersten Anschlüssen 61, 63 des ersten bzw. zweiten Ventilkörpers 60A, 60B einerseits und den zweiten Anschlüssen 62, 64 des ersten bzw. zweiten Ventilkörpers 60A, 60B andererseits hergestellt wird, und in einer zweiten Position (Fig. 6B) eine Strömungsverbindung zwischen dem ersten Anschluss 61 des ersten Ventilkörpers 60A und dem zweiten Anschluss 64 des zweiten Ventilkörpers 60B bzw. dem zweiten Anschluss 62 des ersten Ventilkörpers 60A und dem ersten Anschluss 63 des zweiten Ventilkörpers 60B hergestellt wird. Dadurch kann die Richtung der Strömung durch die Blutbehandlungseinheit, insbesondere die Dialysierflüssigkeitskammer des Dialysators oder Filters, umgekehrt werden. Das Verdrehen der Ventilkörper 60A, 60B erfolgt mit einem nicht dargestellten Drehantrieb, der von der Steuereinheit angesteuert wird.

Die Vorrichtung zur Umkehr der Strömungsrichtung ist in der US 2006/0079 827 A1 im Einzelnen beschrieben. Bei dem vorliegenden Ausführungsbeispiel wird aber die aus der US 2006/0079 827 A1 als solche bekannte Vorrichtung zur Umkehr der Strömungsrichtung auf der Dialysierflüssigkeitsseite verwendet.

## Patentansprüche

1. Vorrichtung zur Überwachung des Anschlusses einer Blutbehandlungseinheit an das Flüssigkeitssystem einer extrakorporalen Blutbehandlungsvorrichtung, wobei die Blutbehandlungseinheit einen Einlass und einen Auslass zum Anschluss an das Flüssigkeitssystem und das Flüssigkeitssystem ein Leitungssystem mit einem ersten Leitungsabschnitt aufweist, der an den Einlass angeschlossen wird, und einen zweiten Leitungsabschnitt aufweist, der an den Auslass der Blutbehandlungseinheit angeschlossen wird, wobei
   die Überwachungsvorrichtung aufweist:

   Mittel (42, 43) zum Messen des Drucks in dem ersten Leitungsabschnitt und/oder zweiten Leitungsabschnitt des Leitungssystems und
   eine die Messwerte der Mittel zum Messen des Drucks in dem ersten und/oder zweiten Leitungsabschnitt auswertende Auswerteinheit (40),

   **dadurch gekennzeichnet, dass**
   die Auswerteinheit (40) derart ausgebildet ist, dass auf der Grundlage des gemessenen Drucks in dem ersten und/oder zweiten Leitungsabschnitt auf einen ordnungsgemäßen oder nicht ordnungsgemäßen Anschluss des Einlasses und Auslasses der Blutbehandlungseinheit an den ersten bzw. zweiten Leitungsabschnitt des Flüssigkeitssystems geschlossen wird.

2. Vorrichtung nach Anspruch 1, **dadurch gekennzeichnet, dass** die Mittel (42, 43) zum Messen des Drucks in dem ersten Leitungsabschnitt und/oder zweiten Leitungsabschnitt des Leitungssystems
   erste Mittel (43) zum Messen des Drucks in dem ersten Leitungsabschnitt des Leitungssystems und
   zweite Mittel (43) zum Messen des Drucks in dem zweiten Leitungsabschnitt des Leitungssystems,
   umfassen, und
   dass die Auswerteinheit (40) derart ausgebildet ist, dass die Differenz $\Delta p = p_1 - p_2$ von dem ersten Druckmesswert und dem zweiten Druckmesswert mit einem vorgegebenen Grenzwert verglichen wird, wobei die Auswerteinheit auf der Grundlage des Vergleichs der Differenz von dem ersten und zweiten Druckmesswert mit dem Grenzwert einen ordnungsgemäßen oder nicht ordnungsgemäßen Anschluss der Blutbehandlungseinheit an das Flüssigkeitssystem feststellt.

3. Vorrichtung nach Anspruch 2, **dadurch gekennzeichnet, dass** die Auswerteinheit derart ausgebildet ist, dass die Auswerteinheit (40) einen nicht ordnungsgemäßen Anschluss der Blutbehandlungseinheit an das Flüssigkeitssystem feststellt, wenn die Differenz $\Delta p = p_1 - p_2$ von dem ersten und zweiten Druckmesswert kleiner als der vorgegebene Grenzwert ist.

4. Vorrichtung nach einem der Ansprüche 1 bis 3, **dadurch gekennzeichnet, dass** die Überwachungsvorrichtung eine Alarmeinheit (45) aufweist, die einen optischen und/oder akustischen und/oder taktilen Alarm gibt, wenn die Auswerteinheit einen nicht ordnungsgemäßen Anschluss der Blutbehandlungseinheit an das Flüssigkeitssystem feststellt.

5. Vorrichtung nach einem der Ansprüche 1 bis 4, **dadurch gekennzeichnet, dass** die Auswerteinheit (40) derart ausgebildet ist, dass der Betrag der Differenz $\Delta p = p_1 - p_2$ von dem ersten Druckmesswert und dem zweiten Druckmesswert berechnet wird, wobei der Betrag der Differenz $\Delta p = p_1 - p_2$ mit dem vorgegebenen Grenzwert verglichen wird.

6. Vorrichtung nach einem Ansprüche 1 bis 5, **dadurch gekennzeichnet, dass** die Auswerteinheit (40) derart ausgebildet ist, dass die Auswerteinheit ein Steuersignal für einen Eingriff in die Maschinensteuerung der Blutbehandlungseinrichtung erzeugt, wenn die Auswerteinheit einen nicht ordnungsgemäßen Anschluss der Blutbehandlungseinheit an das Flüssigkeitssystem feststellt.

7. Extrakorporale Blutbehandlungsvorrichtung mit einer zentralen Steuereinheit (30) und einem Flüssigkeitssystem (II), das ein Leitungssystem mit einem ersten Leitungsabschnitt (10a) aufweist, der an den Einlass (4a) einer Blutbehandlungseinheit (1) angeschlossen wird, und einen zweiten Leitungsabschnitt (12a) aufweist, der an den Auslass (4b) der Blutbehandlungseinheit angeschlossen wird, **dadurch gekennzeichnet, dass** die Blutbehandlungsvorrichtung eine Vorrichtung zur Überwachung des Anschlusses der Blutbehandlungseinheit an das Flüssigkeitssystem der Blutbehandlungsvorrichtung nach einem der Ansprüche 1 bis 4 aufweist.

8. Vorrichtung nach Anspruch 7, **dadurch gekennzeichnet, dass** das Flüssigkeitssystem (II) der Blutbehandlungsvorrichtung ein erstes Absperrorgan (17) zum Absperren des ersten Leitungsabschnitts (10a) und ein zweites Absperrorgan (18) zum Absperren des zweiten Leitungsabschnitts (10b) des Flüssigkeitssystems aufweist, wobei das erste und zweite Absperrorgan von der zentralen Steuereinheit (30) der Blutbehandlungsvorrichtung zum Öffnen und Schließen angesteuert wird.

9. Vorrichtung nach Anspruch 8, **dadurch gekennzeichnet, dass** die ersten Mittel (42) zum Messen des Drucks Mittel (42) zum Messen des Drucks in dem ersten Leitungsabschnitt (10b) stromauf des ersten Absperrorgans (17) und die zweiten Mittel (43) zum Messen des Drucks Mittel zum Messen des Drucks in dem zweiten Leitungsabschnitt (12a) des Leitungssystems stromab des zweiten Absperrorgans (18) sind.

10. Vorrichtung nach einem der Ansprüche 7 bis 9, **dadurch gekennzeichnet, dass** die zentrale Steuereinheit (30) der Blutbehandlungsvorrichtung mit der Auswerteinheit (40) der Überwachungsvorrichtung derart zusammenwirkt, dass nach dem Öffnen des ersten und zweiten Absperrorgans (17, 18) die Messwerte der Mittel (42, 43) zum Messen des Drucks in dem ersten und/oder zweiten Leitungsabschnitt ((10b, 12a) ausgewertet werden.

11. Vorrichtung nach einem der Ansprüche 7 bis 10, **dadurch gekennzeichnet, dass** das Leitungssystem Mittel (50) zum Umkehren der Flussrichtung aufweist, die derart ausgebildet sind, dass in einer ersten Position der erste Leitungsabschnitt (10b) zu dem Einlass (4a) der Blutbehandlungseinheit (1) und der zweite Leitungsabschnitt (12a) zu dem Auslass (4b) der Blutbehandlungseinheit führt, und dass in einer zweiten Position der erste Leitungsabschnitt (10b) zu dem Auslass (4b) der Blutbehandlungseinheit (1) und der zweite Leitungsabschnitt (12a) zu dem Einlass (4a) der Blutbehandlungseinheit führt, wenn die Blutbehandlungseinheit (1) an das Flüssigkeitssystem (II) angeschlossen ist.

12. Vorrichtung nach Anspruch 11, **dadurch gekennzeichnet, dass** die Auswerteinheit (40) derart mit den Mitteln (50) zum Umkehren der Flussrichtung zusammenwirkt, dass die Auswerteinheit (40) die Mittel (50) zum Umkehren der Flussrichtung aktiviert, wenn die Auswerteinheit einen nicht ordnungsgemäßen Anschluss der Blutbehandlungseinheit an das Flüssigkeitssystem feststellt.

13. Vorrichtung nach einem der Ansprüche 7 bis 12, **dadurch gekennzeichnet, dass** die zentrale Steuereinheit (30) der Blutbehandlungsvorrichtung derart ausgebildet ist, dass die zentrale Steuereinheit (30) die Durchführung der Blutbehandlung verhindert, wenn die Auswerteinheit (40) der Überwachungsvorrichtung das Steuersignal zum Eingriff in die Maschinensteuerung erzeugt.

14. Verfahren zum Überwachen des Anschlusses einer Blutbehandlungseinheit an das Flüssigkeitssystem einer extrakorporalen Blutbehandlungsvorrichtung, wobei die Blutbehandlungseinheit einen Einlass und einen Auslass zum Anschluss an das Flüssigkeitssystem und das Flüssigkeitssystem ein Leitungssystem mit einem ersten Leitungsabschnitt aufweist, der an den Einlass angeschlossen wird, und einen zweiten Leitungsabschnitt aufweist, der an den Auslass angeschlossen wird, wobei der Druck in dem ersten Leitungsabschnitt und/oder zweiten Leitungsabschnitt des Leitungssystems gemessen wird,
**dadurch gekennzeichnet, dass**
auf der Grundlage des gemessenen Drucks in dem ersten und/oder zweiten Leitungsabschnitt auf einen ordnungsgemäßen oder nicht ordnungsgemäßen Anschluss des Einlasses und Auslasses der Blutbehandlungseinheit an den ersten bzw. zweiten Leitungsabschnitt des Flüssigkeitssystems geschlossen wird.

15. Verfahren nach Anspruch 14, **dadurch gekennzeichnet, dass** der Druck in dem ersten Leitungsabschnitt des Leitungssystems und in dem zweiten Leitungsabschnitt des Leitungssystems gemessen wird, und dass die Differenz $\Delta p = p_1 - p_2$ von dem ersten Druckmesswert und dem zweiten Druckmesswert mit einem vorgegebenen Grenzwert verglichen wird, wobei auf der Grundlage des Vergleichs der Differenz von dem ersten und zweiten Druckmesswert mit dem Grenzwert ein ordnungsgemäßer oder nicht ordnungsgemäßer Anschluss der Blutbehandlungseinheit an das Flüssigkeitssystem festgestellt wird.

16. Verfahren nach Anspruch 15, **dadurch gekennzeichnet, dass** ein nicht ordnungsgemäßer Anschluss der Blutbehandlungseinheit an das Flüssigkeitssystem festgestellt wird, wenn die Differenz $\Delta p = p_1 - p_2$ von dem ersten und zweiten Druckmesswert kleiner als der vorgegebene Grenzwert ist.

17. Verfahren nach einem der Ansprüche 14 bis 16, **dadurch gekennzeichnet, dass** der Betrag der Differenz $\Delta p = p_1 - p_2$ von dem ersten Druckmesswert und dem zweiten Druckmesswert berechnet wird, wobei dass der Betrag der Differenz $\Delta p = p_1 - p_2$ mit dem vorgegebenen Grenzwert verglichen wird.

18. Verfahren nach einem der Ansprüche 14 bis 17, **dadurch gekennzeichnet, dass** ein optischer und/oder akustischer und/oder taktiler Alarm gegeben wird, wenn ein nicht ordnungsgemäßer Anschluss der Blutbehandlungseinheit an das Flüssigkeitssystem festgestellt wird.

19. Verfahren nach einem der Ansprüche 14 bis 18, **dadurch gekennzeichnet, dass** ein Eingriff in die Maschinensteuerung der Blutbehandlungseinrichtung vorgenommen wird, insbesondere die Durchführung der Blutbehandlung verhindert wird, wenn ein nicht ordnungsgemäßer Anschluss der Blutbehandlungseinheit an das Flüssigkeitssystem festgestellt wird.

20. Verfahren nach einem der Ansprüche 14 bis 19, **dadurch gekennzeichnet, dass** die Flussrichtung zu der Blutbehandlungseinheit umgekehrt wird, wenn ein nicht ordnungsgemäßer Anschluss der Blutbehandlungseinheit an das Flüssigkeitssystem festgestellt wird.

**Claims**

1. Apparatus for monitoring the connection of a blood treatment unit to the fluid system of an extracorporeal blood treatment apparatus, the blood treatment unit comprising an inlet and an outlet for connection to the fluid system, and the fluid system comprising a line system having a first line portion connected to the inlet and a second line portion connected to the outlet of the blood treatment unit, the monitoring apparatus comprising:

   means (42, 43) for measuring the pressure in the first line portion and/or second line portion of the line system, and an evaluation unit (40) which evaluates the measured values from the means for measuring the pressure in the first and/or second line portion,

   **characterised in that**
   the evaluation unit (40) is designed such that a conclusion is drawn, on the basis of the measured pressure in the first and/or second line portion, on whether the inlet and outlet of the blood treatment unit are connected correctly or incorrectly to the first or second line portion of the fluid system, respectively.

2. Apparatus according to claim 1, **characterised in that** the means (42, 43) for measuring the pressure in the first line portion and/or second line portion of the line system comprise first means (43) for measuring the pressure in the first line portion of the line system and second means (43) for measuring the pressure in the second line portion of the line system, and **in that** the evaluation unit (40) is designed such that the difference $\Delta p = p_1 - p_2$ between the first measured pressure value and the second measured pressure value is compared with a preset threshold value, the evaluation unit establishing on the basis of the comparison of the difference between the first and second measured pressure value with the threshold value whether the blood treatment unit is connected correctly or incorrectly to the fluid system.

3. Apparatus according to claim 2, **characterised in that** the evaluation unit is designed such that the evaluation unit (40) establishes that the blood treatment unit is connected to the fluid system incorrectly if the difference $\Delta p = p_1 - p_2$ between the first and second measured pressure value is smaller than the preset threshold value.

4.  Apparatus according to any of claims 1 to 3, **characterised in that** the monitoring apparatus comprises an alarm unit (45) which emits a visual and/or acoustic and/or tactile alarm if the evaluation unit establishes that the blood treatment unit is connected to the fluid system incorrectly.

5.  Apparatus according to any of claims 1 to 4, **characterised in that** the evaluation unit (40) is designed such that the magnitude of the difference $\Delta p = p_1 - p_2$ between the first measured pressure value and the second measured pressure value is calculated, the magnitude of the difference $\Delta p = p_1 - p_2$ being compared with the preset threshold value.

6.  Apparatus according to any of claims 1 to 5, **characterised in that** the evaluation unit (40) is designed such that the evaluation unit generates a control signal for intervention in the machine control of the blood treatment device if the evaluation unit establishes that the blood treatment unit is connected to the fluid system incorrectly.

7.  Extracorporeal blood treatment apparatus comprising a central control unit (30) and a fluid system (II) which comprises a line system having a first line portion (10a) connected to the inlet (4a) of a blood treatment unit (1) and a second line portion (12a) connected to the outlet (4b) of the blood treatment unit, **characterised in that** the blood treatment apparatus comprises an apparatus for monitoring the connection of the blood treatment unit to the fluid system of the blood treatment apparatus according to any of claims 1 to 4.

8.  Apparatus according to claim 7, **characterised in that** the fluid system (II) of the blood treatment apparatus comprises a first valve (17) for shutting off the first line portion (10a) and a second valve (18) for shutting off the second line portion (10b) of the fluid system, the first and second valve being actuated to open and close by the central control unit (30) of the blood treatment apparatus.

9.  Apparatus according to claim 8, **characterised in that** the first means (42) for measuring the pressure are means (42) for measuring the pressure in the first line portion (10b) upstream of the first valve (17), and the second means (43) for measuring the pressure are means for measuring the pressure in the second line portion (12a) of the line system downstream of the second valve (18).

10. Apparatus according to any of claims 7 to 9, **characterised in that** the central control unit (30) of the blood treatment apparatus interacts with the evaluation unit (40) of the monitoring apparatus such that, after the first and second valves (17, 18) have opened, the measured values from the means (42, 43) for measuring the pressure in the first and/or second line portion (10b, 12a) are evaluated.

11. Apparatus according to any of claims 7 to 10, **characterised in that** the line system comprises means (50) for reversing the flow direction which are designed such that, in a first position, the first line portion (10b) leads to the inlet (4a) of the blood treatment unit (1) and the second line portion (12a) leads to the outlet (4b) of the blood treatment unit, and **in that**, in a second position, the first line portion (10b) leads to the outlet (4b) of the blood treatment unit (1) and the second line portion (12a) leads to the inlet (4a) of the blood treatment unit when the blood treatment unit (1) is connected to the fluid system (II).

12. Apparatus according to claim 11, **characterised in that** the evaluation unit (40) interacts with the means (50) for reversing the flow direction such that the evaluation unit (40) activates the means (50) for reversing the flow direction if the evaluation unit establishes that the blood treatment unit is connected to the fluid system incorrectly.

13. Apparatus according to any of claims 7 to 12, **characterised in that** the central control unit (30) of the blood treatment apparatus is designed such that the central control unit (30) prevents blood from being treated if the evaluation unit (40) of the monitoring apparatus generates the control signal for intervention in the machine control.

14. Method for monitoring the connection of a blood treatment unit to the fluid system of an extracorporeal blood treatment apparatus, the blood treatment unit comprising an inlet and an outlet for connection to the fluid system, and the fluid system comprising a line system having a first line portion connected to the inlet and a second line portion connected to the outlet, the pressure in the first line portion and/or second line portion of the line system being measured, **characterised in that** a conclusion is drawn, on the basis of the measured pressure in the first and/or second line portion, on whether the inlet and outlet of the blood treatment unit are connected correctly or incorrectly to the first or second line portion of the fluid system, respectively.

15. Method according to claim 14, **characterised in that** the pressure in the first line portion of the line system and in the second line portion of the line system is measured, and **in that** the difference $\Delta p = p_1 - p_2$ between the first measured pressure value and the second measured pressure value is compared with a preset threshold value, it being established on the basis of the comparison of the difference between the first and second measured pressure value with the threshold value whether the blood treatment unit is connected correctly or incorrectly to the fluid system.

16. Method according to claim 15, **characterised in that** it is established that the blood treatment unit is connected to the fluid system incorrectly if the difference $\Delta p = p_1 - p_2$ between the first and second measured pressure value is smaller than the preset threshold value.

17. Method according to any of claims 14 to 16, **characterised in that** the magnitude of the difference $\Delta p = p_1 - p_2$ between the first measured pressure value and the second measured pressure value is calculated, the magnitude of the difference $\Delta p = p_1 - p_2$ being compared with the preset threshold value.

18. Method according to any of claims 14 to 17, **characterised in that** a visual and/or acoustic and/or tactile alarm is emitted if it is established that the blood treatment unit is connected to the fluid system incorrectly.

19. Method according to any of claims 14 to 18, **characterised in that** intervention in the machine control of the blood treatment device is carried out, in particular blood is prevented from being treated, if it is established that the blood treatment unit is connected to the fluid system incorrectly.

20. Method according to any of claims 14 to 19, **characterised in that** the flow direction to the blood treatment unit is reversed if it is established that the blood treatment unit is connected to the fluid system incorrectly.

## Revendications

1. Dispositif servant à surveiller le raccordement d'une unité de traitement de sang au système de liquide d'un dispositif de traitement de sang extracorporel, l'unité de traitement de sang présentant une entrée et une sortie destinées à être raccordées au système de liquide et le système de liquide présentant un système de conduit pourvu d'un premier tronçon de conduit, qui est raccordé à l'entrée, et d'un deuxième tronçon de conduit, qui est raccordé à la sortie de l'unité de traitement de sang,
le dispositif de surveillance présentant :

des moyens (42, 43) servant à mesurer la pression dans le premier tronçon de conduit et/ou dans le deuxième tronçon de conduit du système de conduit, et
une unité d'analyse (40) analysant les valeurs de mesure des moyens servant à mesurer la pression dans le premier et/ou dans le deuxième tronçon de conduit,

**caractérisé en ce**
**que** l'unité d'analyse (40) est réalisée de telle manière que la conclusion sur un raccordement correct ou incorrect de l'entrée et de la sortie de l'unité de traitement de sang au premier ou au deuxième tronçon de conduit du système de liquide est réalisée sur la base de la pression mesurée dans le premier et/ou dans le deuxième tronçon de conduit.

2. Dispositif selon la revendication 1, **caractérisé en ce que** les moyens (42, 43) servant à mesurer la pression dans le premier tronçon de conduit et/ou dans le deuxième tronçon de conduit du système de conduit comprennent des premiers moyens (43) servant à mesurer la pression dans le premier tronçon de conduit du système de conduit et des deuxièmes moyens (43) servant à mesurer la pression dans le deuxième tronçon de conduit du système de conduit, et
**en ce que** l'unité d'analyse (40) est réalisée de telle manière que la différence $\Delta p = p_1 - p_2$ entre la première valeur de mesure de pression et la deuxième valeur de mesure de pression est comparée à une valeur de limite prédéfinie, l'unité d'analyse constatant sur la base de la comparaison de la différence entre la première et la deuxième valeur de mesure de pression à la valeur de limite un raccordement correct ou incorrect de l'unité de traitement de sang au système de liquide.

3. Dispositif selon la revendication 2, **caractérisé en ce que** l'unité d'analyse est réalisée de telle manière que l'unité d'analyse (40) constate un raccordement incorrect de l'unité de traitement de sang au système de liquide quand la différence $\Delta p = p_1 - p_2$ entre la première et la deuxième valeur de mesure de pression est inférieure à la valeur de limite prédéfinie.

4.  Dispositif selon l'une quelconque des revendications 1 à 3, **caractérisé en ce que** le dispositif de surveillance présente une unité d'alarme (45), qui fournit une alarme optique et/ou acoustique et/ou tactile, quand l'unité d'analyse constate un raccordement incorrect de l'unité de traitement de sang au système de liquide.

5.  Dispositif selon l'une quelconque des revendications 1 à 4, **caractérisé en ce que** l'unité d'analyse (40) est réalisée de telle manière que la valeur de la différence $\Delta p = p_1 - p_2$ entre la première valeur de mesure de pression et la deuxième valeur de mesure de pression est calculée, la valeur de la différence $\Delta p = p_1 - p_2$ étant comparée à la valeur de limite prédéfinie.

6.  Dispositif selon l'une quelconque des revendications 1 à 5, **caractérisé en ce que** l'unité d'analyse (40) est réalisée de telle manière que l'unité d'analyse génère un signal de commande pour une intervention dans la commande de machine du système de traitement de sang quand l'unité d'analyse constate un raccordement incorrect de l'unité de traitement de sang au système de liquide.

7.  Dispositif de traitement de sang extracorporel comprenant une unité de commande (30) centrale et un système de liquide (II), qui présente un système de conduit pourvu d'un premier tronçon de conduit (10a), qui est raccordé à l'entrée (4a) d'une unité de traitement de sang (1), et d'un deuxième tronçon de conduit (12a), qui est raccordé à la sortie (4b) de l'unité de traitement de sang, **caractérisé en ce que** le dispositif de traitement de sang présente un dispositif servant à surveiller le raccordement de l'unité de traitement de sang au système de liquide du dispositif de traitement de sang selon l'une quelconque des revendications 1 à 4.

8.  Dispositif selon la revendication 7, **caractérisé en ce que** le système de liquide (II) du dispositif de traitement de sang présente un premier organe de fermeture (17) servant à fermer le premier tronçon de conduit (10a) et un deuxième organe de fermeture (18) servant à fermer le deuxième tronçon de conduit (10b) du système de liquide, le premier et le deuxième organe de fermeture étant pilotés par l'unité de commande (30) centrale du dispositif de traitement de sang pour l'ouverture et la fermeture.

9.  Dispositif selon la revendication 8, **caractérisé en ce que** les premiers moyens (42) servant à mesurer la pression sont des moyens (42) servant à mesurer la pression dans le premier tronçon de conduit (10b) en amont du premier organe de fermeture (17), et **en ce que** les deuxièmes moyens (43) servant à mesurer la pression sont des moyens servant à mesurer la pression dans le deuxième tronçon de conduit (12a) du système de conduit en aval du deuxième organe de fermeture (18).

10. Dispositif selon l'une quelconque des revendications 7 à 9, **caractérisé en ce que** l'unité de commande (30) centrale du dispositif de traitement de sang coopère avec l'unité d'analyse (40) du dispositif de surveillance de telle manière que les valeurs de mesure des moyens (42, 43) servant à mesurer la pression dans le premier et/ou le deuxième tronçon de conduit (10b, 12a) sont analysées après l'ouverture du premier et du deuxième organe de fermeture (17, 18).

11. Dispositif selon l'une quelconque des revendications 7 à 10, **caractérisé en ce que** le système de conduit présente des moyens (50) servant à inverser le sens d'écoulement, lesquels sont réalisés de telle manière que dans une première position, le premier tronçon de conduit (10b) mène à l'entrée (4a) de l'unité de traitement de sang (1) et le deuxième tronçon de conduit (12a) mène à la sortie (4b) de l'unité de traitement de sang, et **en ce que**, dans une deuxième position, le premier tronçon de conduit (10b) mène à la sortie (4b) de l'unité de traitement de sang (1) et le deuxième tronçon de conduit (12a) mène à l'entrée (4a) de l'unité de traitement de sang, quand l'unité de traitement de sang (1) est raccordée au système de liquide (II).

12. Dispositif selon la revendication 11, **caractérisé en ce que** l'unité d'analyse (40) coopère avec les moyens (50) servant à inverser le sens d'écoulement de telle manière que l'unité d'analyse (40) active les moyens (50) servant à inverser le sens d'écoulement quand l'unité d'analyse constate un raccordement incorrect de l'unité de traitement de sang au système de liquide.

13. Dispositif selon l'une quelconque des revendications 7 à 12, **caractérisé en ce que** l'unité de commande (30) centrale du dispositif de traitement de sang est réalisée de telle manière que l'unité de commande (30) centrale empêche l'exécution du traitement de sang quand l'unité d'analyse (40) du dispositif de surveillance génère le signal de commande aux fins de l'intervention dans la commande de machine.

14. Procédé servant à surveiller le raccordement d'une unité de traitement de sang au système de liquide d'un dispositif de traitement de sang extracorporel, l'unité de traitement de sang présentant une, entrée et une sortie destinées au raccordement au système de liquide et le système de liquide présentant un système de conduit pourvu d'un premier tronçon de conduit, qui est raccordé à l'entrée, et d'un deuxième tronçon de conduit, qui est raccordé à la sortie, la pression étant mesurée dans le premier tronçon de conduit et/ou dans le deuxième tronçon de conduit du système de conduit,
**caractérisé en ce**
**que** la conclusion sur un raccordement correct ou incorrect de l'entrée et de la sortie à l'unité de traitement de sang au premier ou au deuxième tronçon de conduit du système de liquide est réalisée sur la base de la pression mesurée dans le premier et/ou le deuxième tronçon de conduit.

15. Procédé selon la revendication 14, **caractérisé en ce que** la pression dans le premier tronçon du système de conduit et dans le deuxième tronçon du système de conduit est mesurée, et **en ce que** la différence $\Delta p = p_1 - p_2$ entre la première valeur de mesure de pression et la deuxième valeur de mesure de pression est comparée à une valeur de limite prédéfinie, un raccordement correct ou incorrect de l'unité de traitement de sang au système de liquide étant constaté sur la base de la comparaison de la différence entre la première et la deuxième valeur de mesure de pression à la valeur de limite.

16. Procédé selon la revendication 15, **caractérisé en ce qu'**un raccordement incorrect de l'unité de traitement de sang au système de liquide est constaté quand la différence $\Delta p = p_1 - p_2$ entre la première et la deuxième valeur de mesure de pression est inférieure à la valeur de limite prédéfinie.

17. Procédé selon l'une quelconque des revendications 14 à 16, **caractérisé en ce que** la valeur de la différence $\Delta p = p_1 - p_2$ entre la première valeur de mesure de pression et la deuxième valeur de mesure de pression est calculée, la valeur de la différence $\Delta p = p_1 - p_2$ étant comparée à la valeur de limite prédéfinie.

18. Procédé selon l'une quelconque des revendications 14 à 17, **caractérisé en ce qu'**une alarme optique et/ou acoustique et/ou tactile est fournie quand un raccordement incorrect de l'unité de traitement de sang au système de liquide est constaté.

19. Procédé selon l'une quelconque des revendications 14 à 18, **caractérisé en ce qu'**une intervention dans la commande de machine du système de traitement de sang est entreprise, en particulier **en ce que** l'exécution du traitement de sang est empêchée quand un raccordement incorrect de l'unité de traitement de sang au système de liquide est constaté.

20. Procédé selon l'une quelconque des revendications 14 à 19, **caractérisé en ce que** le sens d'écoulement est inversé par rapport à l'unité de traitement de sang quand un raccordement incorrect de l'unité de traitement de sang au système de liquide est constaté.

Fig. 1

Fig. 2A

Fig. 2B

Fig. 3A

Fig. 3B

**Fig. 4**

**Fig. 5**

**Fig. 6A**

**Fig. 6B**

**EP 2 558 142 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10201109 C1
- US 20020121471 A1
- US 20050145549 A1
- US 20060079827 A1